(19) Europäisches Patentamt
European Patent Office
Office européen des brevets

(11) Veröffentlichungsnummer: **0 323 637 B1**

(12) **EUROPÄISCHE PATENTSCHRIFT**

(45) Veröffentlichungstag der Patentschrift: **16.12.92**

(51) Int. Cl.[5]: **C07C 233/66**, A01N 37/18, A01N 37/30

(21) Anmeldenummer: **88121828.3**

(22) Anmeldetag: **29.12.88**

Die Akte enthält technische Angaben, die nach dem Eingang der Anmeldung eingereicht wurden und die nicht in dieser Patentschrift enthalten sind.

# (54) L(-) 2,6-Difluorbenzoesäure-N-(1,1,1-trifluor-prop-2-yl)amid.

(30) Priorität: **07.01.88 DE 3800178**

(43) Veröffentlichungstag der Anmeldung:
**12.07.89 Patentblatt 89/28**

(45) Bekanntmachung des Hinweises auf die Patenterteilung:
**16.12.92 Patentblatt 92/51**

(84) Benannte Vertragsstaaten:
**BE CH DE ES FR GB IT LI NL**

(56) Entgegenhaltungen:
**EP-A- 0 226 837**
**EP-A- 0 243 668**
**EP-A- 0 244 613**

**JOURNAL OF ORGANIC CHEMISTRY, Band 27, April 1962, Seiten 1406-1409, American Chemical Society, Washington, D.C., USA; MAYNARD S. RAASCH: "The Chemistry of Sulfur Tetrafluoride. IX. Reaction with Amino Acids in Hydrogen Fluoride."**

(73) Patentinhaber: **BAYER AG**

**W-5090 Leverkusen 1 Bayerwerk(DE)**

(72) Erfinder: **Tarnow, Horst, Dr.**
**Carl-Diem-Weg 6**
**W-4018 Langenfeld(DE)**
Erfinder: **Baasner, Bernd, Dr.**
**Hamberger Strasse 27d**
**W-5090 Leverkusen 3(DE)**
Erfinder: **Homeyer, Bernhard, Dr.**
**Obere Strasse 28**
**W-5090 Leverkusen 3(DE)**
Erfinder: **Hartwig, Jürgen, Dr.**
**Franz-Esser-Strasse 44**
**W-5090 Leverkusen 3(DE)**

EP 0 323 637 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die neue Verbindung L(-) 2,6-Difluorbenzoesäure-N-(1,1,1-trifluor-prop-2-yl)-amid, ein Verfahren zu ihrer Herstellung und ihre Verwendung in Schädlingsbekämpfungsmitteln, insbesondere als Insektizid und Nematizid.

Es ist bekannt, daß bestimmte Phosphorsäureesteramide wie z.B. O-Ethyl-O-(3-methyl-4-methylthiophenyl)-N-isopropyl-phosphorsäureesteramid (Freibezeichnung: Fenamiphos) sehr gute insektizide und nematizide Eigenschaften aufweisen (vergl. US-PS 2 978 479 sowie Pesticide Manual herausgegeben von British Crop Protection Council, 5. Auflage (1977), Seite 262 mit einer Beschreibung des Handelsproduktes Fenamiphos).

Weiterhin ist bekannt, daß das racemische Gemisch der Verbindung 2,6-Difluorbenzoesäure-N-(1,1,1-trifluorprop-2-yl)-amid insektizide und nematizide Eigenschaften aufweist. Siehe dazu die Deutsche Offenlegungsschrift Nr. 3 611 193. Die vorliegende L-(-)-Form der Verbindung 2,6-Difluorbenzoesäure-N-(1,1,1-trifluor-prop-2-yl)-amid hat jedoch demgegenüber überraschende biologische Vorteile.

Es wurde nun die neue Verbindung L(-) 2,6-Difluorbenzoesäure-N-(1,1,1-trifluor-prop-2-yl)-amid der Formel

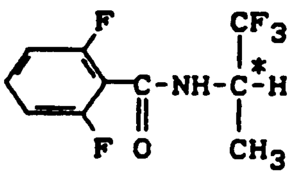

(*L-Form) (I)

gefunden.

Weiterhin wurde gefunden, daß man die neue Verbindung der Formel (I) erhält, wenn man 2,6-Difluorbenzoylhalogenid der Formel (II)

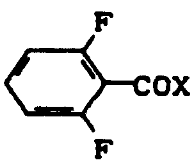

(II)

wobei X = Halogen ist,
mit L(-) 2-Amino-1,1,1-trifluor-propan der Formel (III)

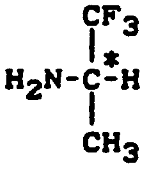

(*L-Form) (III)

bzw. dem entsprechenden Hydrohalogenid, gegebenenfalls in Gegenwart von Säureakzeptoren und gegebenenfalls in Gegenwart von Verbindungsmitteln, umsetzt.

Das neue L(-) 2,6-Difluorbenzoesäure-N-(1,1,1-trifluorprop-2-yl)-amid der Formel (I) zeichnet sich durch eine ausgezeichnete nematizide und insektizide Wirksamkeit und durch eine sehr niedrige Warmblütertoxizität aus.

Die erfindungsgemäße Umsetzung kann durch das folgende Formelschema verdeutlicht werden

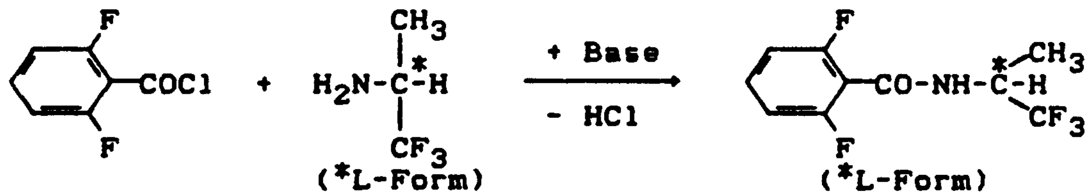


(*L-Form) (*L-Form)

Das als Ausgangsprodukt verwendete 2,6-Difluorbenzoylhalogenid ist durch die Formel (II) allgemein definiert, wobei X vorzugsweise für Chlor und Brom und insbesondere für Chlor steht. Es handelt sich bei den Verbindungen der Formel (II) um bekannte wohlfeile chemische Verbindungen.

Als Beispiele für die Verbindungen der Formel (II) seien genannt: 2,6-Difluorbenzoylchlorid, 2,6-Difluorbenzoylbromid, 2,6-Difluorbenzoylfluorid.

Die beim erfindungsgemäßen Verfahren außerdem als Ausgangsverbindung zu verwendende Verbindung L(-)2-Amino-1,1,1-trifluorpropan der Formel (III) ist neu, wohingegen das entsprechende Racemat bekannt ist (siehe dazu z.B. Deutsche Offenlegungsschrift Nr. 3 611 193).

Das neue Zwischenprodukt der Formel

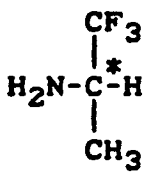

(*L-Form) (III)

kann wie folgt hergestellt werden:

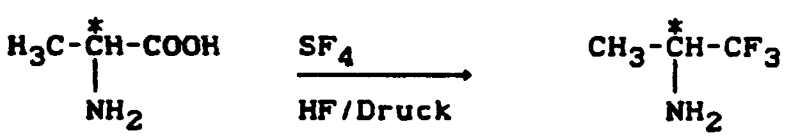


L(+)-Alanin (IV) (L-Form) (III)

Die Umsetzung von L(+)-Alanin mit Schwefeltetrafluorid zur Einführung der Trifluormethylgruppe erfolgt nach einem an sich bekannten Verfahren in wasserfreiem Fluorwasserstoff (siehe dazu Journal of Organic Chemistry 27, 1406 (1962)).

Dazu wird L-(+)-Alanin in einem Stahlrührautoklaven in Fluorwasserstoff mit Schwefeltetrafluorid bei 100 °C bis 180 °C, bevorzugt bei 120 °C bis 160 °C, unter Eigendruck etwa 4 bis 12 Stunden, bevorzugt 6 bis 10 Stunden lang umgesetzt. Pro Mol L-(+)-Alanin werden 2 bis 10 Mol, bevorzugt 2 bis 5, besonders bevorzugt 2 bis 3 Mol Schwefeltetrafluorid sowie 3 bis 200, bevorzugt 3 bis 100, ganz besonders bevorzugt 3 bis 60 Mol Fluorwasserstoff eingesetzt. Zur Aufarbeitung des Umsetzungsgemisches werden die üblicherweise angewandten Verfahren benutzt. So kann man die flüchtigen Bestandteile abdestillieren, das Rohrprodukt mit wäßriger Alkalilauge alkalisch stellen und das Produkt daraus durch Destillation, Extraktion oder Wasserdampfdestillation abtrennen.

Wird beispielsweise das Amin der Formel (III) durch Wasserdampfdestillation abgetrennt, kann das Produkt aus dem Destillat, sofern es sich als zweite Phase abscheidet, durch Phasentrennung isoliert werden. Das Destillat kann aber auch mit Salzsäure angesäuert werden. Durch Einengen bis zur Trockne wird dann das Hydrochlorid des Amins der Formel (III) in reiner Form erhalten. Daraus kann das freie Amin der allgemeinen Formel (III) durch Behandlung mit einer Lauge zur Neutralisation der Salzsäure aus dem Hydrochlorid gewonnen werden. Das Amin wird durch Abdestillieren aus diesem Rohgemisch in reiner Form gewonnen. Es ist aber auch möglich, das Amin der Formel (III) aus diesem Rohgemisch mit einem inerten organischen Lösungsmittel zu extraktieren und das Amin aus diesem Extrakt durch Destillation zu

isolieren.

Beim L-(+)-Alanin (ebenso wie beim D-(-)-Alanin) handelt es sich um ein seit langem bekanntes, wohlfeiles Produkt.

Das erfindungsgemäße Verfahren zur Herstellung der neuen Verbindung der Formel (I) wird vorzugsweise unter Verwendung von Verdünnungsmitteln durchgeführt. Als Verdünnungsmittel kommen dabei Wasser und praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren zur Herstellung der Verbindung (I) alle üblicherweise für derartige Umsetzungen verwendbaren Säurebindemittel eingesetzt werden. Vorzugsweise in Frage kommen Alkalimetallhydroxide wie z.B. Natrium-und Kaliumhydroxid, Erdalkalihydroxide wie z.B. Calciumhydroxid, Alkalicarbonate und -alkoholate wie Natrium-und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Chinolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO). Man kann auch die Amine der Formel (III) im Überschuß einsetzen und den Amin-Überschuß als Säurebinder wirken lassen.

Beim erfindungsgemäßen Verfahren arbeitet man bei Temperaturen zwischen 0 °C und 100 °C, vorzugsweise bei Temperaturen zwischen 20 °C und 80 °C.

Die Umsetzungen werden im allgemeinen unter Normaldruck durchgeführt. Die Umsetzung kann aber auch in geschlossenen Gefäßen und unter erhöhtem Druck vorgenommen werden.

Bei der Durchführung des erfindungsgemäßen Verfahrens setzt man auf 1 Rest -CO-Hal im Benzoylhalogenid der Formel (II) 1,00 bis 1,6 Mol, vorzugsweise 1,05 bis 1,3 Mol Amin der Formel (III) und 1 bis 3 Mol, vorzugsweise 1 bis 2 Mol Säureakzeptor oder ein weiteres Mol des Amins der Formel (III) ein. Die Aufarbeitung des Reaktionsgemisches erfolgt nach üblichen Methoden. Die Verbindung der Formel (I) fällt in fester Form an oder läßt sich - gegebenenfalls nach Einengen der Reaktionslösungen - durch Wasserzugabe abscheiden.

Der Wirkstoff der Formel (I) eignet sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Der Wirstoff (I) ist gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B., Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus,

Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, cheimatobia brumata, Lithocolletis blancardella, Hyponomeuta padella, Plutella maculipennis, Malacosoma neustria, Euproctis chrysorrhoea, Lymantria spp. Bucculatrix thruberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodop tera spp., Trichoplusia ni, Carpocapsa pomonella,Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Der Wirkstoff der Formel (I) kann in due üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen des Wirkstoffs (I) mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,

Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigments, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff (I) kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikrooganismen hergestellte Stoffe u.a.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben dargelegt, weist die erfindungsgemäße neue Verbindung der Formel (I) gegenüber entsprechenden vorbekannten Verbindungen in überraschender Weise eine besonders günstige Warmblütertoxizität und gleichzeitig eine sehr gute Wirksamkeit gegen tierische Schädlinge auf. Diese Eigenschaften erleichtert in vielen Fällen die Anwendung des Wirkstoffs erheblich, insbesondere in Fällen, wo bei der Ausbringung nicht ausreichend erfahrenes Personal zur Verfügung steht und die Einhaltung der bei der Schädlingsbekämpfung erforderlichen und üblichen Vorsichtsmaßnahmen und Sorgfalt nicht voll gewährleistet ist.

Beispiel A

Grenzkonzentrations-Test

Testnematode: Globodera rostochiensis
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18 °C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0%, wenn der Befall genau so hoch ist wie bei den Kontrollpflanzen in unbehandeltem, aber in gleicher Weise verseuchtem Boden.

Die Verbindung gemäß Beispiel 1 zeigte bei diesem Test in einer Konzentration von 1,25 ppm einen Wirkungsgrad von 95 %, wohingegen der gleiche Wirkungsgrad beim aus der DE-OS 3 611 193 bekannten Racemat erst bei einer Konzentration von 10 ppm Racemats 2,6-Difluorbenzoesäure-N-(1,1,1-trifluor-prop-2-yl)-amid eintrat.

Beispiel B

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27 ° C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Die Verbindung gemäß Beispiel 1 zeigte bei diesem Test in einer Konzentration von 1,25 ppm einen Wirkungsgrad von 95 %, wohingegen der gleiche Wirkungsgrad beim aus der DE-OS 3 611 193 bekannten Racemat erst bei einer Konzentration von 10 ppm des Racemats 2,6-Difluorbenzoesäure-N-(1,1,1-trifluor-prop-2-yl)-amid eintrat.

## Herstellungsbeispiele

### Beispiel 1

L(-) 2,6-Difluorbenzoesäure-N-(1,1,1-triflour-prop-2-yl)-amid

17,65 g (0,1 Mol) 2,6-Difluorbenzoylchlorid, gelöst in 50 ml Toluol, werden bei 20 bis 30 ° C zu einer Lösung von 11,30 g (0,1 Mol) 2-Amino-1,1,1-trifluorpropan (L-Form) und 13,50 g (0,1 Mol) N,N-Dimethylbenzylamin in 200 ml Toluol getropft und bei 50 bis 60 ° C gerührt, wobei N,N-Dimethylbenzylamin-Hydrochlorid ausfällt. Anschließend wird auf 20 ° C abgekühlt, mit Wasser versetzt und das feste Reaktionsprodukt abgesaugt. Der Rückstand wird mit Wasser gewaschen und getrocknet.

Man erhält 22,35 g (88,3 % der Theorie) L-(-) 2,6-Difluorbenzoesäure-N-(1,1,1-trifluor-prop-2-yl)-amid vom Schmelzpunkt 178 bis 179 ° C.

### Beispiel 2

2-Amino-1,1,1-trifluorpropan-Hydrochlorid, L-Form

100 g (1,12 Mol) L-Alanin werden mit 280 g Schwefeltetrafluorid und 140 ml HF 8 Stunden lang in einem $V_4$A-Rührautoklaven bei 120 ° C unter Eigendruck (ca. 21 bar) umgesetzt. Nach Abdestillieren der flüchtigen Bestandteile wird der Rückstand in 500 ml Wasser aufgenommen, mit 45 %iger Natronlauge alkalisch gestellt und das Produkt anschließend durch Wasserdampfdestillation abgetrennt. Das Wasserdampfdestillat wird mit konzentrierter Salzsäure sauer gestellt. Nach Einengen und Trocknung werden 80 g (47,6 %) 2-Amino-1,1,1-trifluorpropan als Hydrochlorid in der L-Form isoliert.

### Beispiel 3

(-)-2-Amino-1,1,1-trifluorpropan, L-Form

Zu 59,8 g (0,4 Mol) 2-Amino-1,1,1-trifluorpropan-Hydrochlorid aus Beispiel 2 werden 40 g (0,45 Mol) 45 %ige wäßrige Natronlauge bei 75 bis 80 ° C Ölbadtemperatur in ca. 15 Minuten getropft. Gleichzeitig wird das freie Amin bei 42 bis 48 ° C abdestilliert. Nach Trocknung über Magnesiumsulfat wird redestilliert. Man erhält an Produkt:

41,1 g (91 %) L(-)-2-Amino-1,1,1-trifluorpropan,
Kp.: 47-48 ° C, $n_D^{20}$ : 1,3215
Reinheit nach Gaschromatogramm: 100 %,
$[\alpha]_D^{23}$ : - 9,712(unverdünnt).

**Patentansprüche**

1. L(-) 2,6-Difluorbenzoesäure-N-(1,1,1-trifluor-prop-2-yl)-amid.

2. Verfahren zur Herstellung von L(-) 2,6-Difluorbenzoesäure-N-(1,1,1-trifluor-prop-2-yl)-amid, dadurch gekennzeichnet, daß man 2,6-Difluorbenzoylhalogenid der Formel (II)

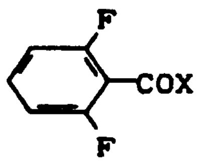

(II)

wobei X für Halogen steht,
mit L(-) 2-Amino-1,1,1-trifluorpropan oder dem entsprechenden Hydrohalogenid, gegebenenfalls in Gegenwart von Säureakzeptoren und gegebenenfalls in Gegenwart von Verdünnungsmitteln umsetzt.

3. Schädlingsbekämpfungsmittel, gekennzeichnet durch einen Gehalt an L(-)2,6-Difluorbenzoesäure-N-(1,1,1-trifluor-prop-2-yl)-amid.

4. Insektizide und nematizide Mittel, gekennzeichnet durch einen Gehalt an L(-)2,6-Difluorbenzoesäure-N-(1,1,1-trifluor-prop-2-yl)-amid.

5. Verfahren zur Bekämpfung von Insekten und/oder Nematoden, dadurch gekennzeichnet, daß man L(-) 2,6-Difluorbenzoesäure-N-(1,1,1-trifluor-prop-2-yl)-amid auf Insekten und/oder Nematoden und/oder deren Lebensraum einwirken läßt.

6. Verwendung von L(-)2,6-Difluorbenzoesäure-N-(1,1,1-trifluor-prop-2-yl)-amid zur Bekämpfung von tierischen Schädlingen, insbesondere von Insekten und Nematoden.

7. Verfahren zur Herstellung von Schädlingsbekämpfungsmitteln, dadurch gekennzeichnet, daß man L(-)2,6-Difluorbenzoesäure-N-(1,1,1-trifluor-prop-2-yl)-amid mit Streckmitteln und/oder oberflächenaktiven Mitteln vermischt.

8. L(-) 2-Amino-1,1,1-trifluorpropan.

9. Verfahren zur Herstellung von L(-)2-Amino-1,1,1-trifluorpropan, dadurch gekennzeichnet, daß man L(+)-Alanin mit Schwefeltetrafluorid in wasserfreiem Fluorwasserstoff unter Druck bei 100°C bis 180°C umsetzt und das Reaktionsprodukt nach an sich bekannten Methoden aufarbeitet.

**Claims**

1. L(-) 2,6-Difluoro-N-(1,1,1-trifluoro-prop-2-yl)-benzamide.

2. Process for the preparation of L(-) 2,6-difluoro-N-(1,1,1-trifluoro-prop-2-yl)benzamide, characterised in that 2,6-difluorobenzoyl halide of the formula (II)

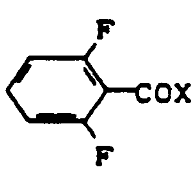

(II)

in which X represents halogen,
is reacted with L(-) 2-amino-1,1,1-trifluoropropane or the corresponding hydrohalide, if appropriate in the

presence of acid acceptors and if appropriate in the presence of diluents.

3. Pesticides, characterised in that they contain L(-)2,6-difluoro-N-(1,1,1-trifluoro-prop-2-yl)-benzamide.

4. Insecticidal and nematicidal agents, characterised in that they contain L(-) 2,6-difluoro-N-(1,1,1-trifluoro-prop-2-yl)-benzamide.

5. Method for controlling insects and/or nematodes, characterised in that L(-) 2,6-difluoro-N-(1,1,1-trifluoro-prop-2-yl)-benzamide is allowed to act on insects and/or nematodes and/or their habitat.

6. Use of L(-)2,6-difluoro-N-(1,1,1-trifluoro-prop-2-yl)-benzamide for controlling animal pests, in particular insects and nematodes.

7. Process for preparing pesticides, characterised in that L(-)2,6-difluoro-N-(1,1,1-trifluoro-prop-2-yl)-benzamide is mixed with extenders and/or surface-active agents.

8. L(-) 2-Amino-1,1,1-trifluoropropane.

9. Process for preparing L(-)2-amino-1,1,1-trifluoropropane, characterised in that L(+)-alanine is reacted with sulphur tetrafluoride in anhydrous hydrogen fluoride at 100°C to 180°C and under pressure, and the reaction product is worked up by methods known per se.

## Revendications

1. L(-) N-(1,1,1-trifluoro-prop-2-yl)-2,6-difluorobenzamide.

2. Procédé pour la fabrication du L(-) 2,6-N-(1,1,1-trifluoro-prop-2-yl)-2,6-difluorobenzamide, caractérisé en ce que l'on fait réagir un halogénure de 2,6-difluorobenzoyle de formule (II)

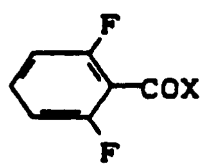

dans laquelle X représente un halogène,
avec du L(-) 2-amino-1,1,1-trifluoro-propane ou avec l'hydrohalogénure correspondant, éventuellement en présence d'accepteurs d'acides et eventuellement en présence de diluants.

3. Agents pour la lutte contre les parasites, caractérisé en ce qu'ils contiennent du L(-) N-(1,1,1-trifluoro-prop-2-yl)-2,6-difluorobenzamide.

4. Agents insecticides et nématicides, caractérisés en ce qu'ils contiennent du L(-) N-(1,1,1-trifluoro-prop-2-yl)-2,6-difluorobenzamide.

5. Procédé pour la lutte contre les insectes et/ou les nématodes, caractérisé en ce qu'on fait agir le L(-) N-(1,1,1-trifluoro-prop-2-yl)-2,6-difluorobenzamide sur les insectes et/ou les nématodes.

6. Utilisation de L(-) N-(1,1,1-trifluoro-prop-2-yl)-2,6-difluorobenzamide pour lutter contre les parasites animaux, en particulier les insectes et les nématodes.

7. Procédé pour la fabrication de produits pour la lutte contre les parasites, caractérisé en ce que l'on mélange du L(-) N-(1,1,1-trifluoro-prop-2-yl)-2,6-difluorobenzamide avec des agents d'allongement et/ou des agents tensioactifs.

8. L(-) 2 amino-1,1,1-trifluoropropane.

**9.** Procédé pour la fabrication de L(-) 2-amino-1,1,1-trifluoropropane, caractérisé en ce que l'on fait réagir de la L(+) alanine avec du tétrafluorure de soufre sous pression à une température de 100 ° C à 180 ° C et qu'on traite le produit réactionnel selon des méthodes connues en soi.